# EUROPEAN PATENT APPLICATION

(11) **EP 0 523 442 A1**
(43) Date of publication of application: **20.01.1993**
(21) Application number: 92111030.0
(22) Date of filing: 30.06.1992
(51) Int. Cl.: A61M 25/00, A61F 2/06

(54) **Device for the extraction of internal urinary catheters**

(30) Priority: 03.07.1991 ES 9102108 U
(71) Applicant: Alvarez-Vijande Garcia, José Ricardo, E-08015 Barcelona (ES)
(72) Inventor: Alvarez-Vijande Garcia, José Ricardo, E-08015 Barcelona (ES)
(74) Representative: Duran Moya, Luis-Alfonso

(57) **Abstract**

The device comprises a generally straight member (1) with a small diametre, having at one end a loop (2) which features a gap (6) close to the thread-like member of the device and having at the other end a handle (8) for its manual control.

It is to be used for the removal of catheters used in connection with the destruction of solid sediments in the kidney, specially by external means, also called litho-destruction.

## Description

This invention refers to a device for the extraction of internal urinary catheters of the type called "double J", self-static, being used in urinary ducts. These catheters are used in Medicine in applications connected with the destruction of solid sediments in the kidney, specially by external means, also called litho-destruction.

The methods for the external destruction of stony sediments in the kidney have been practised since a long time, having as objective the destruction of the "stones" in the kidney by means of shock waves, this permitting the reduction of the size of bigger accumulations of sediments or "stones" into small fragments which can eliminated through the urinary ducts.

To prevent obstructions of the urinary ducts caused by the fragments accumulating from the destruction of bigger stones, it has been usual to introduce catheters within the urinary ducts, which are aimed at establishing the connection between the kidney and the urine bladder, passing, therefore, along the ureters.

The introduction of said catheters is carried out by endoscopic means, that is, with the assistance of means for the mechanical introduction of the catheters featuring optical monitors. Said means are based on the passage of a long member carrying an optical visor which allows to the medical operator to precisely control the passage of the device until reaching the desired location.

For the extraction of the catheter when it is so desired, it is necessary, according to the previously known techniques, to proceed to a new introduction of the mechanical device along the urinary ducts until reaching the urine bladder. In this way, the catheter can be removed. Alternatively, a fine wire or thread may be attached to the end of the catheter passing along the urinary duct, which wire or thread is left partly lying outside the body. By these means the removal of the catheter may be carried out when the medical operator so desires.

However, the first method using a device with optical visors, shows as drawbacks its painful application for the patient and the relative difficulty of its scarce availability in many medical centers due to its high cost and to the high sterilization requirements needed by such method.

The application of the method comprising wires or threads with their ends overhanging to the outside of the body has also important drawbacks, because this method is uncomfortable for the patient and there is the possibility of undesired removal of the wire by the patient himself and also there is an eventual possibility of infection, because, as the wire or thread overhangs externally, it may constitute a route for the penetration of virus into the urinary bladder, ureter and finally into the kidney.

To find a solution to the above mentioned drawbacks, this invention provides a unique and efficient device aimed at permitting the removal of the catheter from the outside of the body without needing wires or threads overhanging externally. Additionally, the invention shows a great simplicity and it is easily applicable.

Essentially, the extraction device of the present invention comprises a long and slender metalic member with a great elasticity and small diameter, of about some tenths of a millimetre, having a specially designed end portion to permit its easy introduction along the urinary ducts, which end portion has a special curved pattern for its attachment to the end of the catheter, preventing any possibility of inflicting damages to the urinary ducts on occasion of its introduction or extraction.

The other end portion or lower end of the device has the form of a handle of small dimensions to allow its simple and efficient manual control.

Given the anatomic characteristics of women, the device of this invention will be preferably applicable to femenine patients, because women have shorter ureteral ducts, which facilitates the introduction of the device within the urine bladder.

For its better understanding, exemplary drawings will be attached corresponding to a preferred embodiment of the device according to this invention. The drawings are aimed at explaining the invention, not to impose limitations on the same.

Figure 1 shows a manual device according to the present invention.

Figure 2 shows a cross section of the ureter conduct, urine bladder and kidney, showing the application of a catheter.

Figure 3 shows a figure similar to figure 2, showing the application of a special catheter.

Figures 4, 5 and 6 are views showing different stages of application of the device for the removal of a catheter.

Figure 7 shows an embodiment of the extraction device with a section adopting an obtuse angle structure to easy up the removal of catheters with pig tail ends.

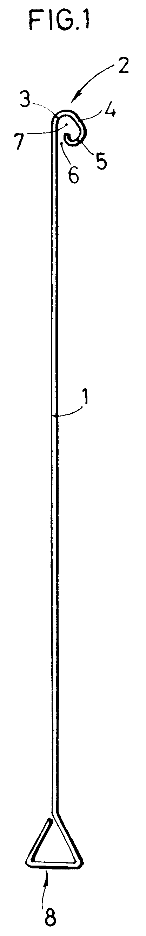

As shown in figure 1, the extraction device which makes the subject of this invention comprises a long and slender body -1-made out of a tough and elastic material, as a plastic material or a special steel, with very reduced thickness and preferably circular cross section. Such device comprises an introduction and recovery portion -2- with a rounded tip -3-, having a slightly diverging side -4- which ends in its lower part in a curved section -5-, with an intermediate gap -6- being left with respect to the member -1-, limiting the opening -7-. At the other end, the device has a handle -8- preferably constituted by successively bending of the elongated member -1- or by means of a ring or similar structure.

Preferably, the introduction head -2- will be formed by wire sections comprised on the same plane.

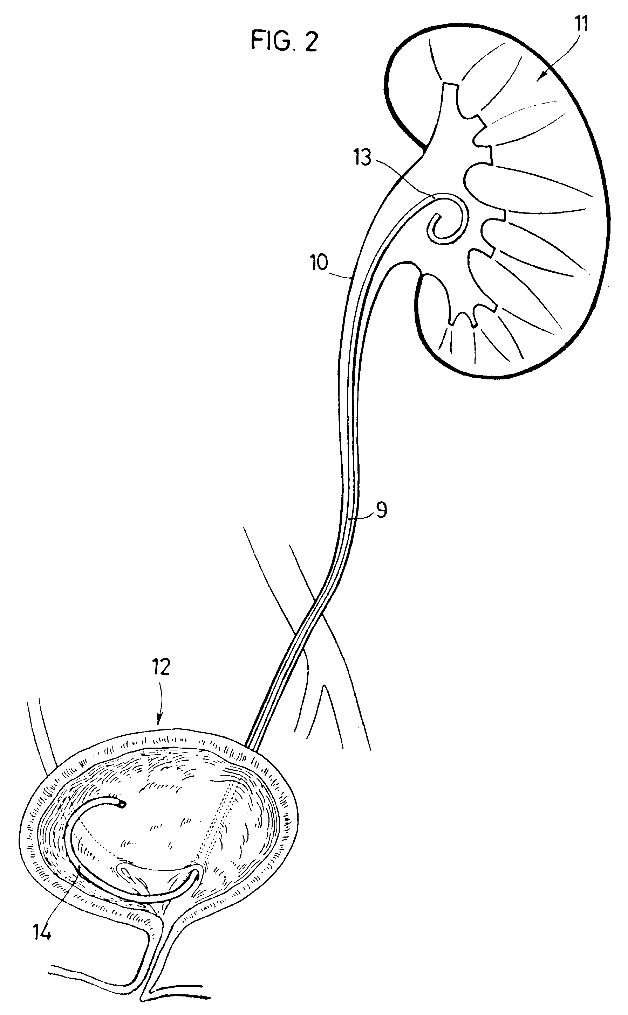

As shown in figure 2, a catheter -9- will pass along the ureter duct -10-, connecting the kidney -11- and the urine bladder -12-. The catheter -9- has in the end which corresponds to the kidney a loop -13- and a second loop -14- in the part which corresponds to the urine bladder.

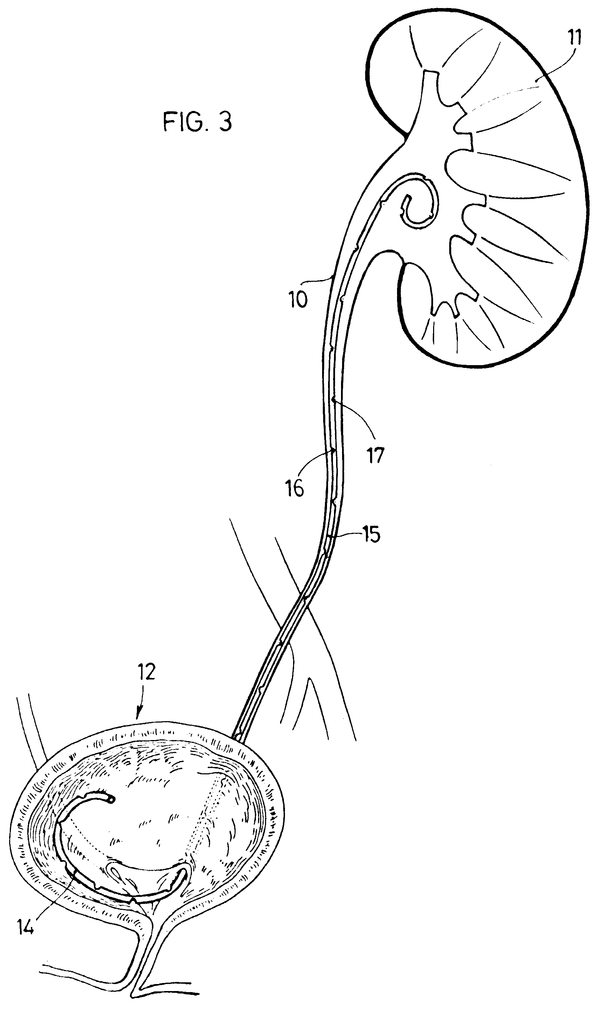

Alternatively, a catheter -15-, shown in figure 3, could be used. This embodiment has a series of recesses such as -16- and -17- distributed along its whole length.

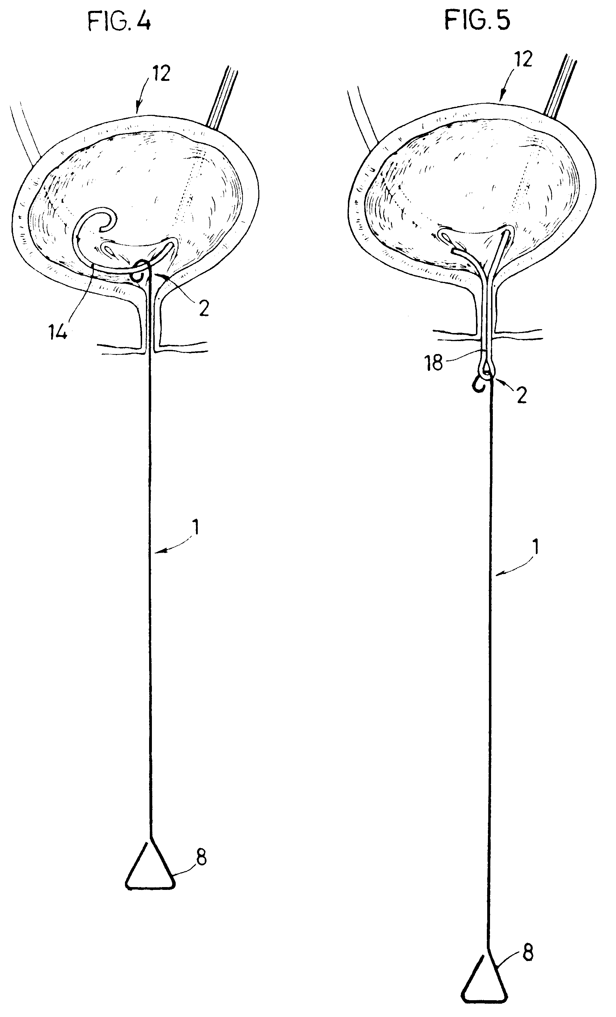

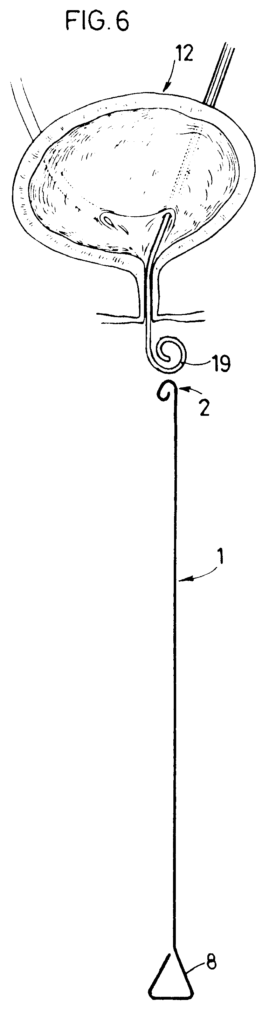

The device -1- will be used as shown in figures 4 to 6, in which said device -1- appears with its introduction head inside of the urine bladder -12-, so that the end loop retains the loop -14- of the catheter, this being made easier by the structure of the introduction head -2-, which allows its introduction and, afterwards, by means of a pulling action, it allows the easy introduction of the loop -14- through the gap -6-. As the pulling action continues on the member -1- the catheter will be removed from the urinary duct forming a half-closed loop -18-, figure 5, after which the extraction will be continued until the end -19- of the catheter will come outside of the urinary duct, allowing the catheter to be removed by hand.

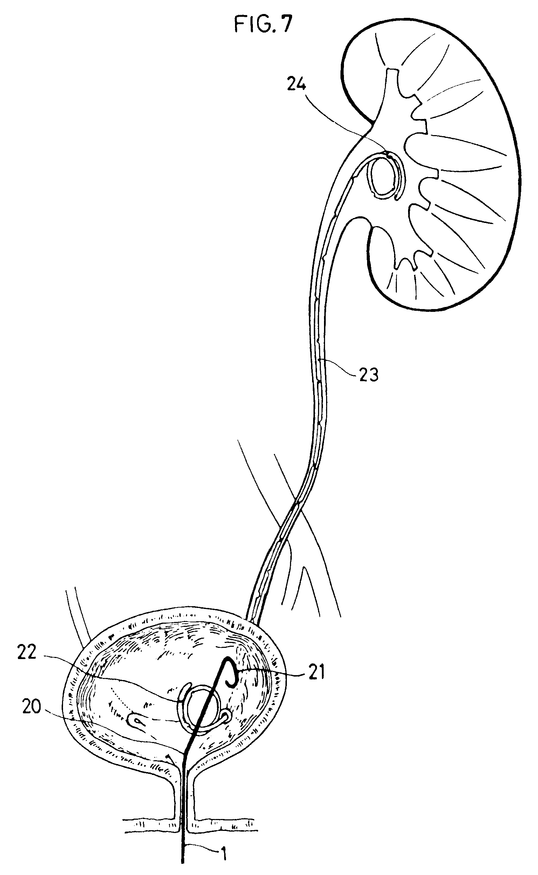

As shown in figure 7, a preferred embodiment of extraction device -1- will have a section with an obtuse angle structure -20- in the vicinity of the end in which the device has the removal loop -21-. This embodiment is specially adequate for the case in which the catheters -23- have pig tail ends -22- and -24-, which show a double loop or helical structure. This arrangement allows the easy removal of this kind of catheters.

Everything which does not alter, change or modify the essentiallity of the described extraction device will be deemed as variable within the scope of this invention.

## Claims

1. Device for the extraction of internal urinary catheters, characterized by a generally straight member with a small diametre, having at one end a loop which features a gap close to the thread-like member of the device and having at the other end a handle for its manual control.

2. Device for the extraction of internal urinary catheters, according to claim 1, characterized in that the end loop has a pointed pattern with a rounded apex, with the end remote from the apex, curved towards the internal part of the loop, leaving an intermediate gap with respect to the thread-like member.

3. Device for the extraction of internal urinary catheters, characterized in that the thread-like member has in the vicinity of its upper end an obtuse angle structure to easy up the removal of catheters with pig tail ends.
